(19) Europäisches Patentamt
European Patent Office
Office européen des brevets

(11) Veröffentlichungsnummer: **0 252 284**
**B1**

(12) # EUROPÄISCHE PATENTSCHRIFT

(45) Veröffentlichungstag der Patentschrift: **06.12.89**

(51) Int. Cl.⁴: **C07C 43/305,** C25B 3/02

(21) Anmeldenummer: **87107943.0**

(22) Anmeldetag: **02.06.87**

(54) **2,6-Dimethyl-p-benzochinontetraalkylketale.**

(30) Priorität: **11.06.86 DE 3619656**

(43) Veröffentlichungstag der Anmeldung: **13.01.88 Patentblatt 88/2**

(45) Bekanntmachung des Hinweises auf die Patenterteilung: **06.12.89 Patentblatt 89/49**

(84) Benannte Vertragsstaaten: **BE CH DE FR GB IT LI NL**

(56) Entgegenhaltungen:
**EP-A- 0 053 261**
**FR-A- 2 295 138**

**JOURNAL OF THE CHEMICAL SOCIETY, PERKIN TRANSACTIONS 1, Nr. 7, 1978, Seiten 681-788; A. NILSSON et al.: "Anodic functionalisation in synthesis. Part 1. Methoxylation of methyl-substituted benzene and anisole derivatives, and the synthesis of aromatic aldehydes by anodic oxidation" Canadian broadcast telidon system": 63-68 (Jan. 31-Feb. 1, 1984) 000**

(73) Patentinhaber: **BASF Aktiengesellschaft, Carl-Bosch-Strasse 38, D-6700 Ludwigshafen(DE)**

(72) Erfinder: **Degner, Dieter, Dr., Kurpfalzstrasse 8, D-6701 Dannstadt-Schauernheim(DE)**
Erfinder: **Guthmann, Alfred, Dr., Duerkheimer Strasse 33, D-6520 Worms 1(DE)**
Erfinder: **Schuster, Ludwig, Dr., Weinheimer Strasse 44, D-6703 Limburgerhof(DE)**
Erfinder: **Hannebaum, Heinz, Ostring 56, D-6700 Ludwigshafen(DE)**

**EP 0 252 284 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue 2,6-Dimethyl-p-benzochinontetraalkylketale.

Aus der EP-A 0 053 261 ist bekannt, daß man Ketale des 2,3,6-Trimethyl-1,4-benzochinons durch elektrochemische Oxidation von 1-Methoxi-2,3,6-trimethylbenzol in Gegenwart von Alkanolen, wie Methanol herstellen kann. Diese Ketale sind Vorprodukte für die Herstellung von Vitamin E. Daß man Anisol durch anodische Oxidation in Gegenwart von Methanol in das Tetramethylketal des p-Benzochinons überführen kann, ist aus J. of Chemical Society, Perkin Trans I, Nr. 7, 1978, Seiten 708–714 bekannt.

Das Tetramethylketal des p-Benzochinon läßt sich auch nach den Angaben der FR-A 2 295 138 durch anodische Oxidation von Benzol in Methanol herstellen. Bei der entsprechenden Elektrolyse von m-Kresylmethylether wird 2-Methyl-p-benzochinontetramethylketal erhalten.

Es wurden nun neue Dimethyl-p-benzochinon-tetraalkylketale mit vorteilhaften Eigenschaften gefunden. Die neuen Verbindungen dieser Erfindung sind 2,6-Dimethyl-p-benzochinontetraalkylketale der Formel

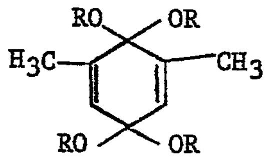

(I),

in der R einen Methyl- oder Ethylrest bedeutet. Diese neuen 2,6-Dimethyl-p-benzochinontetraalkylketale sind Depotverbindungen des toxischen 2,6-Dimethyl-p-benzochinons, das als Stabilisator für Polyesterharze benötigt wird. Weiterhin sind sie Vorprodukte für 2,6-Dimethylhydrochinon, das als Antioxidans verwendet wird.

Die 2,6-Dimethyl-p-benzochinontetraalkylketale der Formel I lassen sich besonders vorteilhaft dadurch herstellen, daß man 2,6-Dimethylphenyl-alkylether der Formel

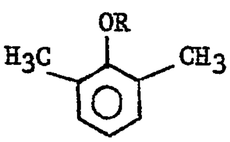

(II),

in der R für einen Methyl- oder Ethylrest steht, in Gegenwart eines Alkanols der Formel ROH, in der R die genannte Bedeutung hat, elektro-chemisch oxidiert.

Die als Ausgangsstoffe benötigten 2,6-Dimethylphenylmethyl- bzw. -ethylether der Formel II lassen sich auf einfache Weise, z.B. durch Umsetzung von 2,6-Dimethylphenol mit Methyl- bzw. Ethylsulfat herstellen. Sie können direkt ohne zusätzliche Reinigung zur Elektrolyse eingesetzt werden.

Die Elektrooxidation benötigt keine spezielle Elektrolysezelle, sie kann in geteilten und ungeteilten Zellen durchgeführt werden. Bevorzugt elektrolysiert man in einer ungeteilten Durchflußzelle. Als Anodenmaterialien werden z.B. Edelmetalle, wie Platin oder Metalloxide, wie $RuO_2$ eingesetzt. Bevorzugtes Anodenmaterial ist Graphit. Als Kathoden werden z.B. solche aus Fe, Ni, Stahl oder Edelmetallen, wie Platin verwendet. Bevorzugt ist auch hier Graphit.

Der Elektrolyt besteht aus einer Lösung von 2,6-Dimethylphenylalkylether in Methanol bzw. Ethanol, die ein Leitsalz enthält. Als Leitsalze lassen sich alle Salze einsetzen, die unter Elektrolysebedingungen zumindest weitgehend stabil sind. Geeignete Leitsalze sind z.B. Sulfate, bzw. Alkylsulfate, wie Tetramethylammoniummethosulfat, Sulfonate, wie $KSO_3C_6H_5$, Fluoride, wie NaF und KF, Tetrafluoroborate, wie $NaBF_4$, Hexafluorophosphate, wie $KPF_6$ und Alkoholate, wie $NaOCH_3$. Weiterhin lassen sich auch Basen, wie KOH verwenden.

Der Elektrolyt hat beispielsweise folgende Zusammensetzung:
5 - 49 Gew.-% 2,6-Dimethylphenyl-alkylether
50 - 90 Gew.-% Methanol bzw. Ethanol
0,1 - 6 Gew.-% Leitsalze

Die Stromdichten liegen bei dem erfindungsgemäßen Verfahren zwischen 1 und 25 A/dm², vorzugsweise wird bei 2 bis 6 A/dm² gearbeitet. Das Verfahren wird bevorzugt drucklos durchgeführt. Man elektrolysiert zweckmäßigerweise bei Temperaturen bis etwa 5°C unterhalb des Siedepunkts von Methanol bzw. Ethanol. Die Elektrolyse kann sowohl diskontinuierlich als auch kontinuierlich durchgeführt werden.

Die Aufarbeitung der Elektrolyseausträge erfolgt nach konventionellen Methoden, vorzugsweise wird destillativ aufgearbeitet. Überschüssiges Methanol(Ethanol), Leitsalz, unumgesetzter 2,6-Dimethylphenylalkylether sowie der als Zwischenstufe durchlaufene 2,6-Dimethylhydrochinondialkylether können zur Elektrolyse zurückgeführt werden.

Beispiel:

| | |
|---|---|
| Apparatur | ungeteilte Zelle mit 11 Elektroden |
| Anode | Graphit |
| Elektrolyt | 330 g 2,6-Dimethylanisol (15%) |
| | 22 g KF (1%) |
| | 1 848 g Methanol (84%) |
| Kathode | Graphit |
| Stromdichte | 3,3 $A/dm^2$ |
| Temperatur | 20 bis 25°C |

Elektrolyse mit 6 F/Mol 2,6-Dimethylanisol.
Der Elektrolyt wird während der Elektrolyse mit 200 l/h über einen Wärmetauscher gepumpt.

Aufarbeitung:

Nach Beendigung der Elektrolyse wird Methanol bei Normaldruck abdestilliert, das ausgefallene Leitsalz (KF, 21 g) abfiltriert und das Filtrat dann bei 70 bis 130°C im Vakuum (Kopfdruck: 2 mbar) reindestilliert. Hierbei erhält man 5,2 g unumgesetztes 2,6-Dimethylanisol (0,038 Mol), 20,6 g 2,6-Dimethylhydrochinondimethylether (0,125 Mol) und 366,5 g 2,6-Dimethyl-p-benzochinontetramethylketale [1,607 Mol, Fp. 29°C, $^1$HNMR ($CDCl_3$) : δ :1,8 ($-CH_3$), 3,0, 3,3 ($-OCH_3$), 6,1 (=CH-)]
Hieraus errechnen sich:

| | | |
|---|---|---|
| Umsatz | 2,6-Dimethylanisol | 98,4% |
| Ausbeute | 2,6-Dimethyl-p-benzochinontetramethylketal | 66,3% |
| Selektivität | 2,6-Dimethyl-p-benzochinontetramethylketal | 71,0% |

## Patentansprüche

1. 2,6-Dimethyl-p-benzochinontetraalkylketale der Formel

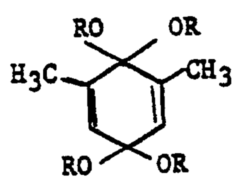

(I)

in der R einen Methyl- oder Ethylrest bedeutet.
2. 2,6-Dimethyl-p-benzochinontetramethylketal gemäß Anspruch 1.
3. 2,6-Dimethyl-p-benzochinontetraethylketal gemäß Anspruch 1.

## Claims

1. A 2,6-dimethyl-p-benzoquinone tetraalkyl ketal of the formula

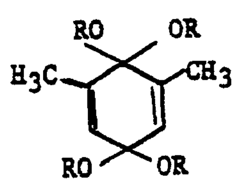

(I)

where R is methyl or ethyl.
2. 2,6-Dimethyl-p-benzoquinone tetramethyl ketal, as encompassed in claim 1.
3. 2,6-dimethyl-p-benzoquinone tetraethyl ketal, as encompassed in claim 1.

## Revendications

1. 2,6-Dimethyl-p-benzoquinonetetraalkylcétals de formule

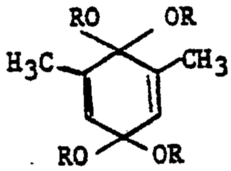

(I)

dans laquelle R représente un reste méthyle ou éthyle.

2. 2,6-Diméthyl-p-benzoquinonetétraméthylcétal selon la revendication 1.

3. 2-6-Diméthyl-p-benzoquinonetétraéthylcétal selon la revendication 1.